# EUROPEAN PATENT APPLICATION

(11) **EP 4 521 210 A1**
(43) Date of publication of application: **12.03.2025**
(21) Application number: 24199483.9
(22) Date of filing: 10.09.2024
(51) Int. Cl.: G06F 3/01, G06F 1/16

(54) **ELECTRODES FOR PHYSIOLOGICAL SIGNAL RECOGNITION**

(30) Priority: 11.09.2023 US 202363581965 P; 04.09.2024 US 202418824712
(71) Applicant: Apple Inc., Cupertino, CA 95014 (US)
(72) Inventor: SHI, Justin S., Cupertino, 95014 (US); LEE, Chia-Yeh, Cupertino, 95014 (US); DOGRUSOZ, Kaan E., Cupertino, 95014 (US); HA, Hyunsoo, Cupertino, 95014 (US); LEE, Seulki, Cupertino, 95014 (US); FERNANDES, David N., Cupertino, 95014 (US)
(74) Representative: Zacco Denmark A/S

(57) **Abstract**

Electrodes that can be used to determine a first type of physiological signal and a second type of physiological signal are disclosed. The first type of physiological signal can be electromyography (EMG) signals produced by activity of muscles and tendons. Identification of hand movements can be supplemented with pose detection circuitry such as inertial measurement units (IMUs). These EMG signals can be processed to identify hand movements and recognize gestures associated with those hand movements. The second type of physiological signal can be electromyography (ECG) signals produced by the heart. The electrodes can be used to detect EMG and ECG signals dependent upon a current configuration of sensing circuitry. The electrodes can be used to determine a measurement quality associated with the EMG signals.

## Description

### FIELD OF THE DISCLOSURE

This relates generally to gesture recognition and heart rate detection, and more particularly to electrodes used to detect gestures and for heart rate detection.

### BACKGROUND OF THE DISCLOSURE

Many types of input can be provided for performing operations in a computing system, such as buttons or keys, mice, trackballs, joysticks, touch sensor panels, touch screens and the like. In addition, other types of input such as audio input (e.g., voice commands), accelerometer input (e.g., device motion, shaking, etc.) and user gestures can also be provided as inputs. In particular, a person's physical motions, such as eye gaze, body movement and the like can be detected and tracked over time as inputs to a computing system. Hand gestures, in particular, can be detected by touch or proximity sensors in a touch sensing panel. However, these sensors generally have limited detection range, and therefore the hand gestures must be performed in close proximity to the panel. Some computing systems can include circuitry to detect cardiac characteristics of a user of the computing systems. Attempting to integrate circuitry to sense hand gestures in addition to detecting cardiac characteristics often requires computing systems that are complex to manufacture and unwieldy.

### SUMMARY OF THE DISCLOSURE

Examples of the disclosure are directed to electrodes that can be formed in a backside of a device that can be configured to detect posing and movement of a hand of a user of the device and can be configured to detect a heartbeat of the user. In some examples, the device can configure sensing circuitry to measure the electrodes to detect electromyography (EMG) signals, which is the electrical activity that results from the contraction of muscles. In some examples, the device configures the electrodes to detect EMG signals that are produced by activity of the flexor and extensor muscles and tendons in the forearm, wrist, and/or hand of a user. The EMG signals can be routed to processing circuitry in the housing of the wrist-worn device. These EMG signals can be processed to identify hand movements such as hand flexion, extension, pronation, supination, radial deviation, ulnar deviation, finger pinching, and finger movement, and recognize gestures and/or poses associated with those hand movements. In some examples, the device can be configured to detect electrocardiogram (ECG) signals, which is the electrical activity that results from palpitations of a heart of the user. In some examples, the device configures sensing circuitry to measure the electrodes to detect ECG signals produced by activity of the heart of the user. The ECG signals can be routed to processing circuitry housed in the device to determine a heart rate (HR) of the user. In some examples, the device further includes pose detection circuitry such as one or more inertial measurement units (IMUs). The IMUs can be configured to supplement pose and/or movement detection determined using EMG signals. In some examples, the device can detect a quality of a measurement associated with the EMG signals, such as a conductance between electrodes detecting the EMG signals, and/or an amount of noise coupling to the EMG signals. In some examples, when the quality of the measurement is suboptimal, the device can detect pose and/or gestures using signals detected by the pose detection circuitry and forgo consideration of the EMG signals in detecting the pose and/or gestures.

### BRIEF DESCRIPTION OF THE DRAWINGS

FIG. 1 illustrates various hand movements that can be performed as part of a hand gesture according to some examples of the disclosure.
FIG. 2 illustrates various muscles and tendons that can be activated to perform the various hand movements of FIG. 1 according to some examples of the disclosure.
FIG. 3 illustrates an example device in which electrodes can be configured for measuring different types of physiological signals according to some examples of the disclosure.
FIG. 4 illustrates a block diagram of an example computing system that illustrates one implementation of physiological signal capture and processing according to some examples of the disclosure.
FIG. 5A illustrates three representative electrodes and simplified circuitry of a device in a first configuration for measuring a first type of physiological signals according to examples of the disclosure.
FIG. 5B illustrates three representative electrodes and simplified circuitry of a device in a second configuration for measuring a second type of physiological signals according to examples of the disclosure.
FIG. 6 illustrates an example configuration for detecting poses and gestures using one or more types of detection circuitry included in an electronic device according to examples of the disclosure.
FIG. 7 illustrates an example configuration of circuitry configured to detect suboptimal physiological signal measurement conditions according to examples of the disclosure.

### DETAILED DESCRIPTION

In the following description of examples, reference is made to the accompanying drawings which form a part hereof, and in which it is shown by way of illustration specific examples that can be practiced. It is to be understood that other examples can be used and structural changes can be made without departing from the scope of the disclosed examples.

Examples of the disclosure are directed to electrodes that can be formed in a device, such as the backside of the device, that can be configured to detect posing and movement of a hand of a user of the device and can be configured to detect a heartbeat of the user. In some examples, the device can configure sensing circuitry to measure the electrodes to detect electromyography (EMG) signals, which is the electrical activity that results from the contraction of muscles. In some examples, the device configures the electrodes to detect EMG signals that are produced by activity of the flexor and extensor muscles and tendons in the forearm, wrist, and/or hand of a user. The EMG signals can be routed to processing circuitry in the housing of the wrist-worn device. These EMG signals can be processed to identify hand movements such as hand flexion, extension, pronation, supination, radial deviation, ulnar deviation, finger pinching, and finger movement, and recognize gestures and/or poses associated with those hand movements. In some examples, the device can be configured to detect electrocardiogram (ECG) signals, which is the electrical activity that results from palpitations of a heart of the user. In some examples, the device configures sensing circuitry to measure the electrodes to detect ECG signals produced by activity of the heart of the user. The ECG signals can be routed to processing circuitry housed in the device to determine a heart rate of the user. In some examples, the device further includes pose detection circuitry such as one or more inertial measurement units (IMUs). The IMUs can be configured to supplement pose and/or movement detection determined using EMG signals. In some examples, the device can detect a quality of a measurement associated with the EMG signals, such as a conductance between electrodes detecting the EMG signals, and/or an amount of noise coupling to the EMG signals. In some examples, when the quality of the measurement is suboptimal, the device can detect pose and/or gestures using signals detected by the pose detection circuitry and forgo consideration of the EMG signals in detecting the pose and/or gestures.

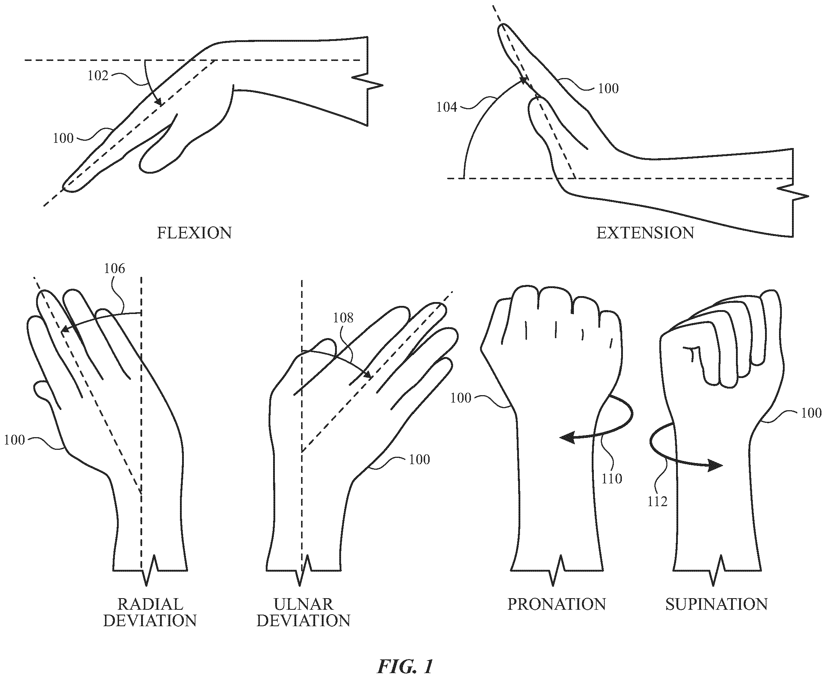

FIG. 1 illustrates various hand movements that can be performed as part of a hand gesture according to some examples of the disclosure. For example, hand 100 can perform one or more of wrist flexion 102, extension 104, radial deviation 106, ulnar deviation 108, pronation 110, or supination 112. Any one of these movements, or a combination of these movements, can be performed as a gesture. In some examples, hand 100 can perform gestures while maintaining a wrist position. For example, hand 100 can perform one or more gestures using one or more fingers. Additionally, hand 100 can maintain one or more gestures performed by one or more fingers and/or the wrist corresponding to hand 100 of a user. In some examples, hand 100 can maintain a pose. For example, hand 100 can maintain a pointing of one or more fingers, a fist pose including a curling of one or more fingers, and/or a pinch pose including contact between one or more fingers.

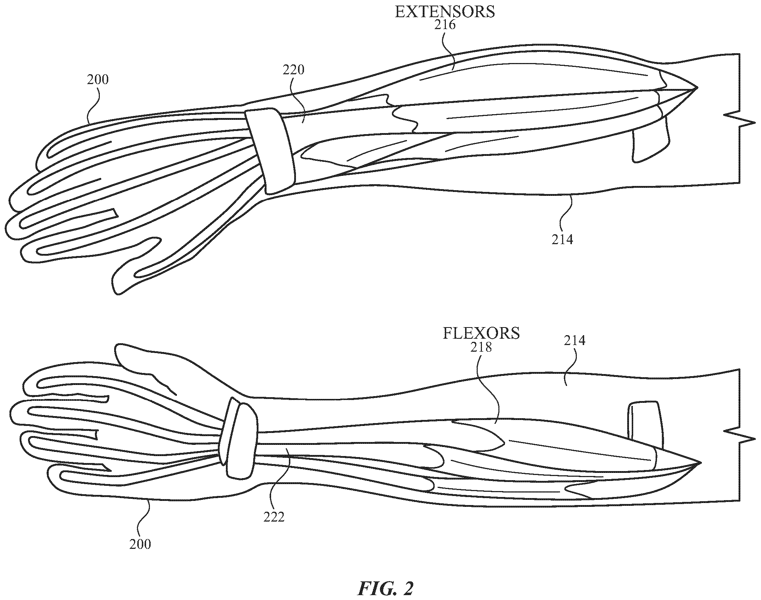

FIG. 2 illustrates various muscles and tendons that can be activated to perform the various hand 200 movements of FIG. 1 according to some examples of the disclosure. Forearm 214 includes forearm extensor muscles 216 and flexor muscles 218, and wrist extensor tendons 220 and flexor tendons 222. When these muscles and tendons contract or extend to perform the hand 200 movements of FIG. 1, electrical signals can be detected. Electromyography (EMG) can be used to measure electrical activity that results from the contraction of extensor muscles 216, flexor muscles 218, extensor tendons 220, and flexor tendons 222. From this electrical activity, various hand movements and gestures shown in FIG. 1 can be detected and used to initiate or perform various functions. Additionally, EMG can be used to detect muscular and tendon movement, posing of one or more fingers and/or an arm of the user, and/or an amount of muscular tension corresponding to the strength of a pose (e.g., a pinching force exerted by muscles forming a pinching gesture).

The wrist is a common and socially acceptable location to house electronics (e.g., smart watches, fitness trackers), and muscles and tendons at this location can provide the electrical activity needed to detect hand movements and identify hand gestures and poses being performed. The wrist is further a common point of physical contact for determining the heart rate of the user. Some examples of the disclosure are directed to devices that are capable of using a shared set of circuitry (e.g., electrodes) to perform both hand gesture and/or pose detection and heart rate detection of the user, thus simplifying a bill-of-materials associated with such devices and consequentially reducing cost and design complexity required to separately include circuitry to perform both such functions.

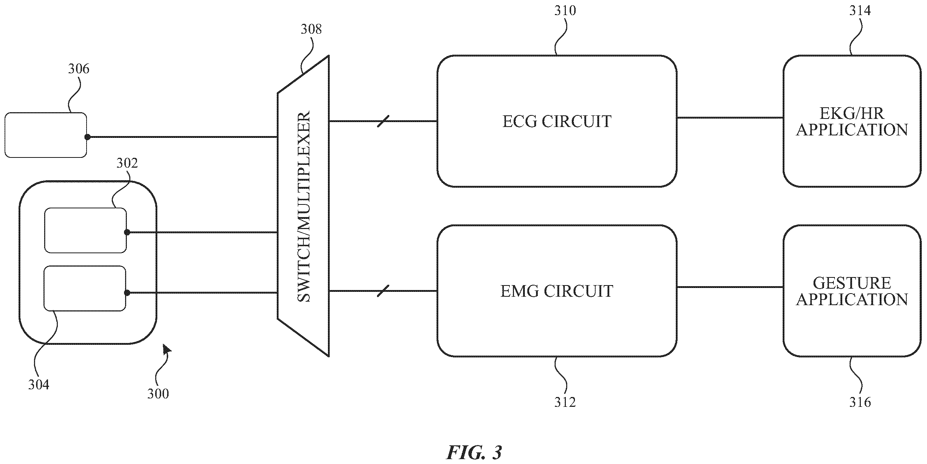

FIG. 3 illustrates an example device in which electrodes can be configured for measuring different types of physiological signals. In some examples, a wearable device includes a plurality of electrodes that can alternatively be configured to measure a first type of physiological signal(s) such as signal(s) used to detect a heart rate of a user of the wearable device during a first period of time, or configured to measure a second type of physiological signal(s) such as signal(s) used to detect one or more gestures of the user. For example, electronic device 300 illustrates a backside of wrist-worn wearable device such as a smartwatch that includes a first electrode - electrode 302 - and a second electrode - electrode 304. In some examples, electrode 302 and/or electrode 304 are "dry" electrodes that can directly contact the skin of the user, without requiring a dielectric medium such as a dielectric gel to improve coupling between electrode 302 and the skin of the user, and/or electrode 304 and the skin of the user. In some examples, as described further herein, characteristic(s) of the electrodes such as a material, size, one or more dimensions, shape, and/or placement of the electrodes are configured to optimize the physiological measurement of multiple types of physiological signals, such as signals associated with determining a heart rate of the user, associated with detecting a gesture performed by the user, and/or to detect maintained poses of portions of the user's body.

In some examples, electronic device 300 includes circuitry and/or software stored in memory circuitry configured to perform one or more operations toggling between a first mode or a second mode of operation. For example, electronic device 300 in the first mode of operation is configured to detect the heart rate of the user. In some examples, electronic device includes a display such as a touch-sensitive display or a non-touch sensitive display embedded in a housing of electronic device 300. In some examples, electronic device 300 is worn on a primary hand of the user. In some examples, electronic device 300 displays a prompt within a user interface via the display, requesting the user contact a location included in electronic device 300, such as a crown 306, with a secondary hand in order to perform an electrocardiogram measurement. FIG. 3 includes the crown 306 as the location included in the electronic device 300, however examples are not so limited. For example, other locations can include a bezel, a dedicated location on a housing of the electronic device 300, a location on a display of the electronic device 300, a band coupled to the electronic device 300, etc. Palpitations of a heart of the user can cause electrical impulses to propagate from the heart outward, thereby causing differences in voltage (e.g., electrical potential) along the skin of the user. In some examples, these electrical potentials can be detected by circuitry such as the electrode 302, 304, and/or the location included in electronic device 300 (e.g., crown 306), and the electrical potentials can be processed and/or analyzed to determine heart rate and other aspects of the user's physiological signals such as an electrocardiogram (ECG). It is understood that examples of the disclosure directed to detecting physiological signals using crown 306 can additionally or alternatively be implemented by detecting physiological signals via one or more of the locations included in electronic device 300 described herein.

In such an example, electronic device 300 configures multiplexer 308 to couple signals differentially between electrode 302 and electrode 304 to ECG circuitry 310, and to couple crown 306 to sensing circuitry (e.g., included in or separate from the ECG circuitry). For example, multiplexer 308 includes one or more switches coupling electrode 302 and/or electrode 304 to one or more amplifiers (e.g., a shared amplifier, such as an instrumentation amplifier), and/or one or more switches coupling crown 306 to circuitry (e.g., a buffer and/or filter). It is understood that ECG circuitry 310 can include one or more amplifiers, passive circuits (e.g., including resistors, capacitors, inductors, and the like), active components (e.g., amplifiers, and/or buffers), analog-to-digital converters (ADCs), an additional or alternative circuitry suitable to furnish electronic device 300 with analog and/or digital signals for further processing to determine the heart rate of the user.

In some examples, in accordance with a determination that the electronic device detects that contact between electrode 302, electrode 304, and/or crown 306 and the user's body satisfies one or more criteria, electronic device 300 proceeds to perform an electrocardiogram (ECG) measurement. For example, the one or more criteria include a criterion satisfied when physical a contact between the wrist of a primary hand is sufficient to obtain a signal with a signal-to-noise ratio (SNR) higher than a first threshold value, and/or including a criterion satisfied when physical contact between one or more body parts such as fingers and/or a palm of a secondary hand are sufficient to obtain a signal with a SNR higher than a second threshold value - the same or different as the first threshold value. If the one or more criteria are satisfied, electronic device 300 proceeds to detect physiological signals to perform an ECG measurement. In some embodiments, the satisfaction of the one or more criteria is mandatory, optional, or completely forgone before electronic device 300 performs the ECG measurement.

To measure a physiological signal, crown 306 can act as a measurement electrode, and electrodes 302 and 304 can act as a reference and/or ground electrodes respectively (or vice-versa) to perform an ECG measurement. For example, physiological characteristics caused by electrical activity of the heart of the user can be measured via crown 306 (e.g., at the secondary hand of the user not wearing electronic device 300). When the user's body is contacting crown 306, a conductive path can be created through a physiological signal source (e.g., the heart of the user and/or other suitable tissues intermediate to the electrical path between crown 306 and electrodes 302 and/or 304).

EKG/HR Application 314 corresponds to software such as one or more instructions stored in memory of electronic device 300 configured to convert one or more ECG measurements to an estimate of the heart rate. In some embodiments, the ECG measurement is performed once or a plurality of times to provide a plurality of measurements of physiological signals to the EKG/HR Application 314 to determine an aggregation of the measurements (e.g., an averaging) to improve confidence of determinations of the user's heart rate. In some examples, one or more signals are discarded in accordance with a determination that such signals correspond to erroneous measurements. In some examples, the one or more signals provided to the EKG/HR Application 314 are used to determine an abnormality in the user's heart rate, such as arrhythmia and/or an abnormally high or low heart rate. Thereafter, the EKG/HR Application 314 can cause display via a display of electronic device 300 indicating such an abnormality, such as displaying of a warning, and/or cause generation of an auditory and/or tactile warning such as a chime, klaxon, and/or one or more vibrations of electronic device 300.

Although the examples and applications of electronic devices and methods are described in the context of generating a complete ECG waveform, it should be understood that the same or similar devices and methods may be used to collect and process data from the plurality of measurement electrodes and may or may not generate an ECG waveform. For example, the signals from electrodes 302 and 304 may facilitate the monitoring of certain cardiac characteristics (e.g., heart rate, arrhythmias, changes due to medications or surgery, function of pacemakers, heart size, etc.) and/or ECG waveform characteristics (e.g., timing of certain waves, intervals, complexes of the ECG waveform) by a digital signal processor without generating a complete ECG waveform. In some examples, the controller may generate a subset of the ECG waveform (e.g., one or more of the P wave, QRS complex, PR interval, T wave, U wave). Moreover, examples of the disclosure include contact detection and processing devices and methods configured for other types of physiological signal measurements including, but not limited to, EEG measurements, EMG measurements and/or optical determination of heart rate.

Turning back to the mode of operations, electronic device 300 can be configured in a second mode of operation to detect one or more gestures and/or poses of a portion of the user's body. For example, electronic device 300 can couple electrode 302 and/or electrode 304 to EMG circuitry 312 instead of ECG circuitry 310 in the second mode of operation via multiplexer 308. It is understood that ECG circuitry 310 and EMG circuitry 312 optionally can include a shared set of circuitry (e.g., can share one or more amplifiers, passive components, analog-to-digital converters, filters, and/or other suitable circuitry), and that coupling to ECG circuitry 310 "instead" of coupling to EMG circuitry 312 can include coupling electrode 302 and/or electrode 304 to at least partially different circuitry (e.g., different amplifiers, passive components, analog-to-digital converters, filters, and/or other suitable circuitry), such as to different signal lines, and vice-versa. During the second mode of operation, EMG circuitry 312 can condition and/or convert physiological signals (e.g., a differential signal between electrode 302 and 304) to provide Gesture Application 316 with one or more signals indicating a current pose and/or movement of a hand of the user, such as the primary hand wearing electronic device 300. The gesture and/or pose detection of electronic device 300 is described further with reference to at least FIG. 2, and further description of such detection is omitted here for brevity. In some examples, crown 306 is not connected, terminated with a load, and/or measurements of crown 306 can be forgone during the second mode of operation. Thus, during the second mode of operation, electronic device 300 can re-use electrode 302 and electrode 304 to perform gesture and/or pose detection of the user. Therefore, electronic device 300 can selectively toggle between heart rate detection and gesture detection using a same set of electrodes 302 and 304 during different modes of operation. Configuring the electrodes to perform two different types of physiological measurements reduces the need for inclusion of additional or alternative electrodes, thus potentially reducing the amount of materials and/or manufacturing complexity required to include multiple electrodes configured to separately carry out the distinct types of physiological measurements.

In some examples, during the second mode of operation, one or more poses and/or gestures performed by the user's body such as the user's primary hand wearing electronic device 300 are determined. Hand movement "signatures" comprised of various expected electrode measurements captured at certain times can be stored or determined, either empirically during product development or as a result of the device being "trained" after the user performs a requested series of hand movements. During actual user hand movement, physiological measurements can be captured over a period of time at electrodes 302 and 304 and compared by a processor included in electronic device 300 to the known hand movement "signatures." If the captured data matches a particular hand movement "signature" or a series of signatures, a hand movement, gesture, and/or a maintained pose can be identified the processor. The number of electrode measurements to be taken can depend on a mode of operation. For example, if only coarse gesture recognition is needed (e.g., only a fist gesture, an open hand gesture, and/or a pinch gesture needs to be detected), only a first set of measurements (e.g., one, two, three, or four measurements), may need to be detected by electronic device 300 to generate three or four channels of data to detect muscular activity that matches the signatures for those gestures. However, if the detection of more gestures, and/or more complex gestures is desired, then additional measurements may need to be monitored.

In some examples, electronic device 300 includes a casing, housing, or watch body including the electrodes 302 and 304, and/or including a device such as a watchband for mechanically coupling the electronic device 300 to the user's body. In some examples, electrodes 302 and/or 304 are embedded in the band, and electrical measurements of physiological signals are coupled and transmitted to electronic device 300. In the example of FIG. 3, one or more wireless communication modules (e.g., a Bluetooth Low Energy radio module, a Zigbee module) may be needed to facilitate transmission and/or reception of electrode signals to a separate device (e.g., a smartphone or other handheld or wearable device) for processing. In some examples, electronic device 300 is a glove 336 (e.g., for AR/VR applications) having a flexible cuff including electrodes 302 and/or 304, electrically and/or communicatively coupled to electronic device 300. It is understood that the precise form of electronic device 300 described herein is merely exemplary, and that additional or alternative devices can be envisaged without departing from the scope of the disclosure.

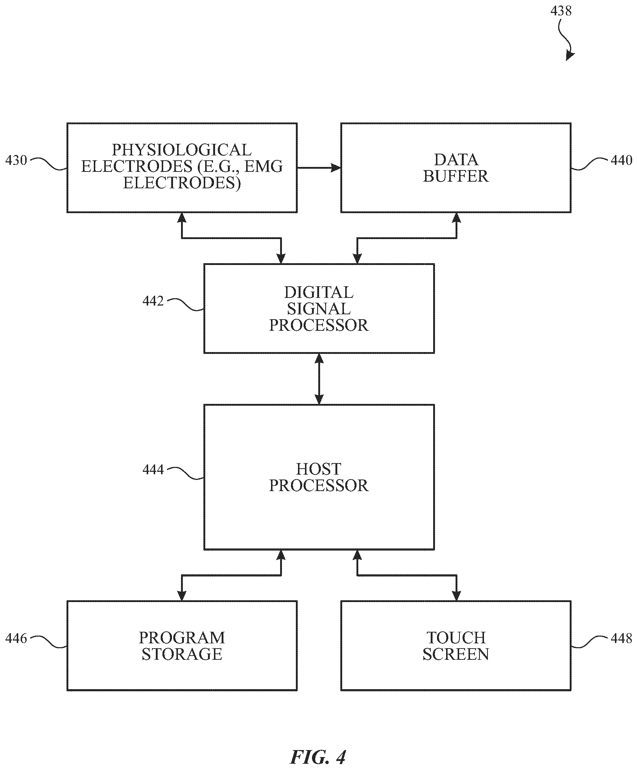

FIG. 4 illustrates a block diagram of an example computing system 438 that illustrates one implementation of physiological signal capture and processing according to some examples of the disclosure. Portions of computing system 438 can be included in, for example, electronic devices 300 and/or any separate mobile or non-mobile, wearable or non-wearable computing device for physiological signal analysis and/or display. Computing system 438 can include one or more physiological electrodes 430 (e.g., EMG/ECG electrodes) to measure electrical signals (e.g., EMG/ECG signals) from a person contacting the EMG/ECG electrodes, data buffer 440 (or other volatile or non-volatile memory or storage) to store temporarily (or permanently) the physiological signals from the physiological electrodes 430, digital signal processor (DSP) 442 to analyze and process the physiological signals, host processor 444, program storage 446, and in some examples, touch screen 448 to perform display operations (e.g., to display real time EMG/ECG signals or provide user alerts). In some examples, touch screen 448 may be replaced by a non-touch sensitive display.

Host processor 444 can be electrically coupled to program storage 446 to execute instructions stored in program storage 446 (e.g., a non-transitory computer-readable storage medium). Host processor 444 can, for example, provide control and data signals to generate a display image on touch screen 448, such as a display image of a user interface (UI). Host processor 444 can also receive outputs from DSP 442 (e.g., an EMG/ECG signal) and performing actions based on the outputs (e.g., display the EMG/ECG signal, play a sound, provide haptic feedback, etc.). Host processor 444 can also receive outputs (touch input) from touch screen 448 (or a touch controller, not-shown). Such outputs from DSP 442 can be used by computer programs stored in program storage 446 to perform actions that can include, but are not limited to, moving an object such as a cursor or pointer, scrolling or panning, adjusting control settings, opening a file or document, viewing a menu, making a selection, executing instructions, operating a peripheral device connected to the host device, answering a telephone call, placing a telephone call, terminating a telephone call, changing the volume or audio settings, storing information related to telephone communications such as addresses, frequently dialed numbers, received calls, missed calls, logging onto a computer or a computer network, permitting authorized individuals access to restricted areas of the computer or computer network, loading a user profile associated with a user's preferred arrangement of the computer desktop, permitting access to web content, launching a particular program, encrypting or decoding a message, and/or the like. It is understood that the output from DSP 442 can be used to perform one or more operations at electronic device 300, and/or at additional or alternative devices in communication with electronic device 300, such as other wearable (e.g., head-mounted) devices, laptop computers, desktops, media set-top boxes, and the like. Host processor 444 can also perform additional functions that may not be related to touch processing and display.

Note that one or more of the functions described herein, including the processing of physiological signals, can be performed by firmware stored in memory (e.g., in DSP 442) and executed by one or more processors (in DSP 442), or stored in program storage 446 and executed by host processor 444. The firmware can also be stored and/or transported within any non-transitory computer-readable storage medium for use by or in connection with an instruction execution system, apparatus, or device, such as a computer-based system, processor-containing system, or other system that can fetch the instructions from the instruction execution system, apparatus, or device and execute the instructions. In the context of this document, a "non-transitory computer-readable storage medium" can be any medium (excluding signals) that can contain or store the program for use by or in connection with the instruction execution system, apparatus, or device. The computer-readable storage medium can include, but is not limited to, an electronic, magnetic, optical, electromagnetic, infrared, or semiconductor system, apparatus or device, a portable computer diskette (magnetic), a random access memory (RAM) (magnetic), a read-only memory (ROM) (magnetic), an erasable programmable read-only memory (EPROM) (magnetic), a portable optical disc such a CD, CD-R, CD-RW, DVD, DVD-R, or DVD-RW, or flash memory such as compact flash cards, secured digital cards, universal serial bus (USB) memory devices, memory sticks, and the like.

The firmware can also be propagated within any transport medium for use by or in connection with an instruction execution system, apparatus, or device, such as a computer-based system, processor-containing system, or other system that can fetch the instructions from the instruction execution system, apparatus, or device and execute the instructions. In the context of this document, a "transport medium" can be any medium that can communicate, propagate or transport the program for use by or in connection with the instruction execution system, apparatus, or device. The transport medium can include, but is not limited to, an electronic, magnetic, optical, electromagnetic or infrared wired or wireless propagation medium.

It is to be understood that the computing system 438 is not limited to the components and configuration of FIG. 4, but can include other or additional components (or omit components) in multiple configurations according to various examples. For example, an analog-to-digital converter (ADC) may be added between physiological electrodes 430 and DSP 442 to convert the signals to the digital domain, or touch screen 448 can be omitted and the EMG/ECG signal or other information from the analysis and processing can be relayed to another device (e.g., a tablet, laptop, smartphone, computer, server, etc.) via wired or wireless connection that can include a display or other feedback mechanism for outputting a visual representation of the data or other notifications or information. Additionally, the components of computing system 438 can be included within a single device, or can be distributed between multiple devices.

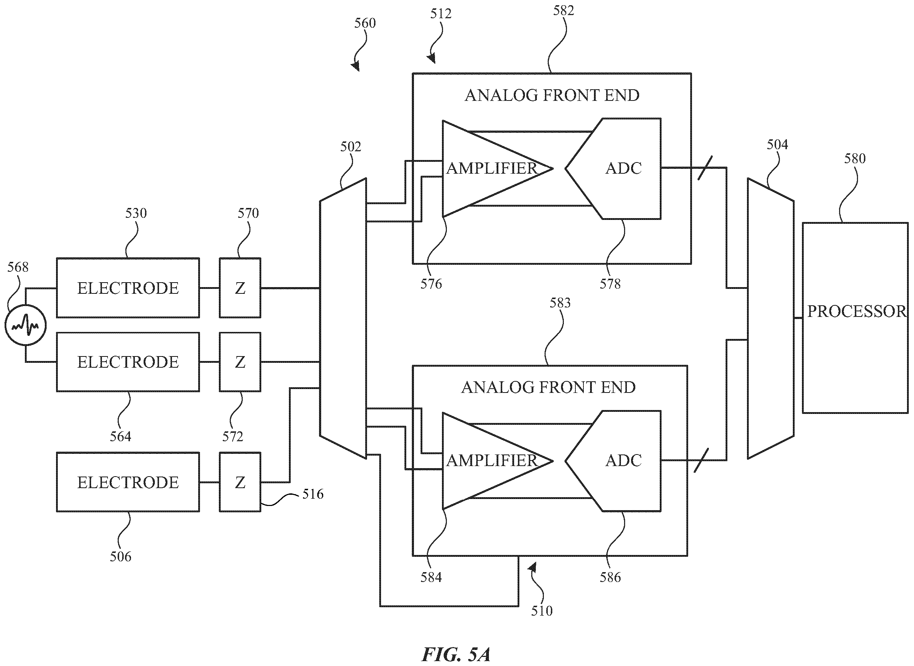

FIG. 5A illustrates three representative electrodes and simplified circuitry of a device in a first configuration for measuring a first type of physiological signals according to examples of the disclosure. In FIG. 5A, differential measurement circuitry 560 can be located in a housing of the device (e.g., electronic device 300 of FIG. 3) and can include processor 580 and analog front end 582 and 583.

In some examples, during a first mode of operation, differential measurement circuitry 560 is configured to detect one or more physiological signals to determinate a heart rate of a user of a device. For example, the user's secondary hand (e.g., the hand not wearing the device) contacts electrode 506 while differential measurement circuitry 560 is configured to operate in the first mode of operation. In the first mode of operation, multiplexer 502 (e.g., corresponding to multiplexer 308 described with respect to FIG. 3 herein) optionally includes one or more switches to couple electrodes 530 and 564 (e.g., corresponding to an electrode included in crown 306, and either electrode 302 or electrode 304, described herein with respect to FIG. 3) to inputs of amplifier 584. Thus, an electrode 530 (e.g., included in crown 306) and electrode 564 (e.g., electrode 302 or 304) are optionally coupled to physiological signal source 568, and are coupled to analog front end 583 (e.g., differentially coupled to amplifier 584). To effect such a configuration, multiplexer 502 optionally also includes one or more switches configured to couple ground electrode 506 (e.g., corresponding to electrode 304 or 302 that is not configured as electrode 564) to analog front end 583 (e.g., to a voltage reference of amplifier 584). In some examples, electrode 530 is coupled to multiplexer 502 via network 570, and electrode 564 is coupled to multiplexer 502 via network 572. Networks 570 and 572 represent the equivalent circuits of the signal paths between electrode 530 and amplifier 576, or between electrode 564 and the amplifier, respectively. In some examples, networks 570 and 572 can include circuit components (e.g., resistors, capacitors, inductors, amplifiers and/or buffers, and/or diodes) and/or can include impedances inherent in the signal paths (e.g., routing impedances, parasitic impedances, etc.). In some examples, networks 570 and 572 can provide electrostatic discharge (ESD) protection for differential measurement circuitry 560 and/or provide safety by limiting or preventing electrical currents being applied to the user's skin and/or preventing unexpected or unintentional external signals from entering the device and causing damage. Networks 570 and 572 thus are configured to condition signals indicative of physiological measurements provided by physiological signal source 568, feeding signals back to amplifier 584.

In some examples, analog front end 583 includes amplifier 584 and analog-to-digital converter (ADC) 586. Electrocardiogram (ECG) waveforms can be generated based on the electrical activity of the heart during each heartbeat, measured differentially between electrode 530 and electrode 564. In some examples, amplifier 584 can output an amplified differential signal and ADC 586 can convert the amplified differential signal into a digital signal. In some examples, amplifier 584 can output an amplified single-ended output. In some examples, the output of ADC 586 can be a multi-bit signal (e.g., 8 bits, 12 bits, 24 bits, etc.) electrically coupled to processor 580. The multi-bit signal can be transmitted from analog front end 583 to processor 580 serially or in parallel. In some examples, ADC 586 can be a differential ADC that converts a differential analog input to a digital output. In some examples, ADC 586 can be single-ended ADC that converts a single-ended analog input to a digital output. In some examples, differential amplifier 584 can be implemented with two single-ended amplifiers and ADC 586 can be implemented with two ADCs (each electrically coupled to the output of one of the single-ended amplifiers). In the first mode of operation, multiplexer 504 is configured to couple one or more outputs of ADC 586 to processor 580 for further processing to determine a heart rate of the user, such as via one or more switches.

As described above, electrode 530 and electrode 564 can come into contact with the body of the user. When electrode 530 and electrode 564 contact a user's body (e.g., contact a portion of a secondary hand and/or a wrist of a primary hand, respectively), the electrodes can receive a first type of physiological signal - indicative of a heart rate - from the user. In FIG. 5A, the first type of physiological signal is represented as physiological signal source 568. In some examples, when the user touches electrode 530, a path can be created through physiological signal source 568 from electrode 530 to electrode 564. In some examples, contacting electrode 530 can cause differential measurement circuitry 560 to measure a physiological signal from physiological signal source 568, which is thereafter used to determine the heart rate of the user as described further with reference to FIG. 3.

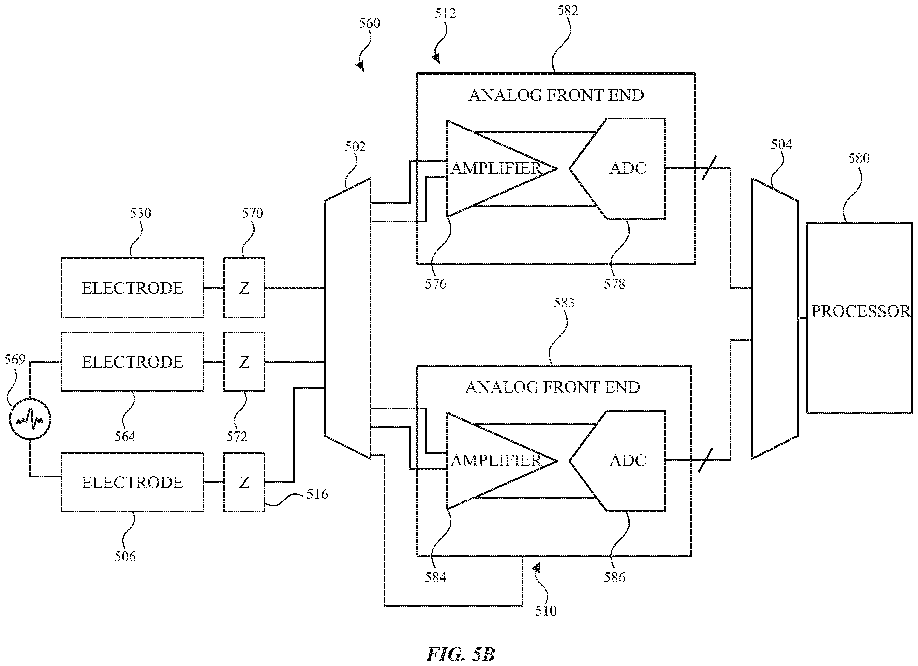

FIG. 5B illustrates three representative electrodes and simplified circuitry of a device in a second configuration for measuring a second type of physiological signals according to examples of the disclosure. In some examples, during a second mode of operation, differential measurement circuitry 560 is configured in a second configuration to detect one or more physiological signals indicative of maintained pose and/or gesture of one or more body parts of the user of the device. For example, in the second mode of operation, physiological source 569 - representative of electrical impulses caused by movement of tendons, muscles, and/or other tissue in a hand and/or arm of a user - is coupled to electrodes 564 and 506. Electrodes 564 and 506 as described previously, optionally correspond to electrodes 302 and 304 of FIG. 3. In such an example, electrode 530 is optionally not used (e.g., disconnected, terminated with a load, and/or ignored for purposes of EMG determinations). In some examples, electrode 506 is coupled to multiplexer 502 via network 516, having one or more characteristics similar or the same as those described with reference to networks 570 and/or 572. In some examples, electrode 564 is also coupled to multiplexer 502, similar or the same as described with reference to the first mode of operation. In the contrast with the first mode of operation, however, multiplexer 502 is configured to provide a signal path from electrode 564 to amplifier 576 via one or more switches included in multiplexer 502 when configured in the second configuration. In some examples, electrode 564 and 506 are configured as differential inputs to amplifier 576, similar or the same as previously described with reference to electrodes coupled to amplifier 584 as previously described with respect to FIG. 5A. In such examples, analog front end 582 provides amplification of one or more outputs from amplifier 576, thus amplifying one or more physiological signals indicative of movement and/or posing of the user. Similar to ADC 586, ADC 578 is coupled to an amplifier (e.g., amplifier 576), and optionally quantizes the analog output from the amplifier, providing a digital representation of the value of the analog output to multiplexer 504. During the second mode of operation, multiplexer 504 is configured to couple one or more outputs of ADC 578 to processor 580, thus furnishing processor 580 with digital measurements of the user's movement and/or pose. In some examples, the differential measurement circuitry 560 is configured to share an ADC between outputs of the amplifier 584 and 576. For example, multiplexer 504 optionally is coupled to the outputs of the amplifiers 584 and 576, and selectively couples the output of amplifier 584 or 576 to the shared ADC when operating in the first mode or the second mode of operation.

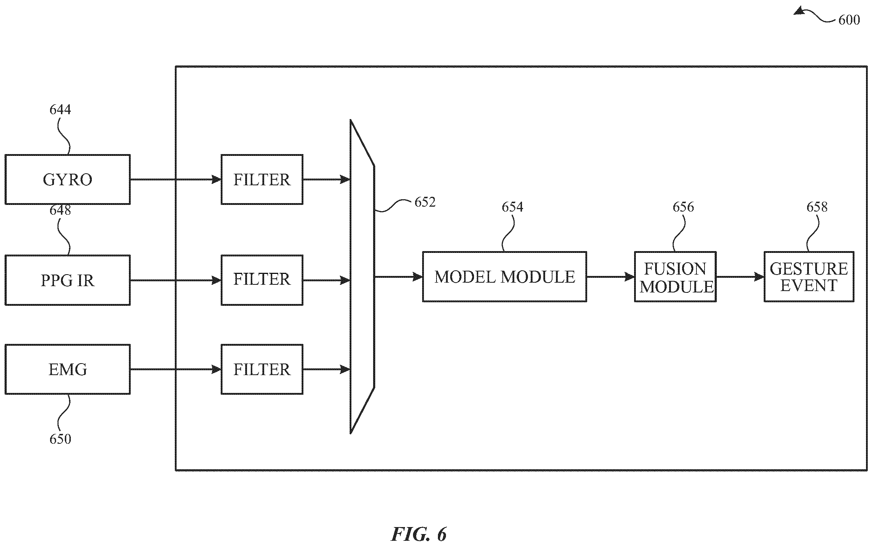

FIG. 6 illustrates an example configuration for detecting poses and gestures using one or more types of detection circuitry included in an electronic device according to some examples of the disclosure. In some examples, an electronic device (e.g., a wearable electronic device) can be configured to use one or more types of pose and gesture detection circuitry, alone or in some combination, to detect poses and gestures of the user's body. For example, electronic device 600 - corresponding to circuitry and/or software and/or instructions included in electronic device 300 of FIG. 3 - includes a plurality of gesture detection modules. It is understood that gesture detection modules can refer to circuitry and/or software instructions configured to detect poses and gestures. A gyroscopic module 644, for example, optionally includes one or more gyroscopes configured to detect an orientation and/or movement of electronic device 600 and/or housing of the electronic device. In some examples, gyroscopic module 644 includes one or more microelectromechanical systems (MEMS) devices (e.g., accelerometers, magnetometers, and/or gyroscopes, such as one or more inertial measurement units (IMUs)) configured to detect movement and/or orientation of electronic device 600. In some examples, movement of electronic device, such as translation of the device, rotation along one or more axes of the device, and/or maintenance of the electronic device at a particular orientation and/or position can be detected using gyroscopic module 644, providing information and/or data for further processing. The information and/or data obtained from the gyroscopic module 644 is provided via one or more data channels to software included in electronic device 600, such as to a model module 654. In some examples, as described further herein, model module 654 includes one or more algorithmic and/or machine learning models configured to process and/or refine data associated with movement and/or posing of the user's body (e.g., arm, hand, and/or finger) wearing electronic device 600. In some examples, a plurality of model modules are included in electronic device 600. For example, gyroscopic module 644, optical module 648, and/or EMG module 650 respectively are communicatively coupled to respective model modules. The respective model modules can have characteristic(s) similar or the same as described with reference to model module 654. Additionally, the respective model modules can be configured to detect, condition, process, analyze, and/or otherwise manipulate information and/or data received by their corresponding gesture detection modules (e.g., gyroscopic module 644, optical module 648, and/or EMG module 650). For example, the respective model modules are optimized to perform operations based on characteristics of data and/or information obtained from their corresponding gesture detection modules, such as optimized to perform operations with respect to EMG data obtained from EMG module 650, but not necessarily optimized to perform operations with respect to optical module 648.

In some examples, electronic device 600 includes optical circuitry configured to detect characteristics of the user's vasculature. For example, optical module 648 is optionally representative of circuitry configured to perform a plethysmogram. As an example, optical module 648 optionally includes one or more light sources (e.g., light-emitting diodes (LEDs)) configured to generate light, and/or to detect transmissive absorption and/or reflection incident upon tissues of the user. Such one or more light sources can be used to detect pulse oximetry, in addition or as an alternative to detecting estimations of the heart rate of the user. In some examples, the optical module 648 optionally uses the one or more light sources to detect indications of movement, such as blood flowing in and out of tissues in response to contraction of muscles and/or tendons of the user's body. In some examples, similar to as described with reference to gyroscopic module 644, electronic device 600 detects signals via the optical module 648, and converts the signals to information and/or data that is furnished to model module 654.

In some examples, electronic device 600 includes EMG module 650. EMG module includes one or more circuits and/or circuitry described with reference to FIGs. 3-5B. Further description of EMG circuitry is omitted for brevity.

In some examples, electronic device 600 communicates information and/or data obtained via gyroscopic module 644, optical module 648, and/or EMG module 650 to model module 654, via one or more data channels respectively configured to carry data to model module 654 from the modules, and through multiplexer 652. For example, multiplexer 652 can be representative of hardware switching circuitry and/or software including instructions configured to select or combine signals and/or data received via the one or more data channels and provide data to model module 654. Model module 654 can be stored in memory of electronic device 600, and can include instructions to perform operations (e.g., run algorithms analyzing the data, and/or applying one or machine learning models) in accordance with the data from the various modules described herein.

In some examples, electronic device 600 applies information and/or data obtained from the model module 654 to a fusion module 656. Fusion module 656, similar to model module 654, can be stored in memory of electronic device 600, and can include instructions to perform operations (e.g., run algorithms analyzing the data, and/or applying one or machine learning models) to synthesize information and data indicating movement and/or posing of the user's body. For example, fusion module 656 can be configured to estimate a kinematic state of the user's body. As an example, fusion module 656 can use data and/or information implicated by model module 654 as indicative of the user's pose and movement to determine a previous, current, and/or possible state of the user's arm, hand, and/or fingers. In some examples, fusion module 656 can use one or more Karlman filters to recursively identify a current pose and/or gesture in view of a previously identified pose and/or gesture. Additionally or alternatively, fusion module 656 can use one or more machine learning and/or deep learning models, including but not limited to Bayesian networks, convolutional neural networks (CNNs), and/or other suitable techniques to ascertain a relative weighting and/or confidence in one or more sources of data (e.g., the modules described herein) in determining a current and/or future pose and/or movement of the user's body. For example, EMG data can be discarded when it is determined that the measurement environment to obtain EMG data is suboptimal, as described further below. In some examples, fusion module 656 provides information and/or data suggestive of the current and/or future pose and/or movement of electronic device 600 to gesture event module 658. Gesture event module 658 can use such information and/or data to perform one or more operations described further herein (e.g., navigating a user interface, answering a call, causing movement of a cursor, etc.). In some examples, model module 654 and fusion module 656 are combined into a single model module. It is understood that such a combined model module can have one or more characteristics that are similar or the same as those described with reference to the constituent modules herein.

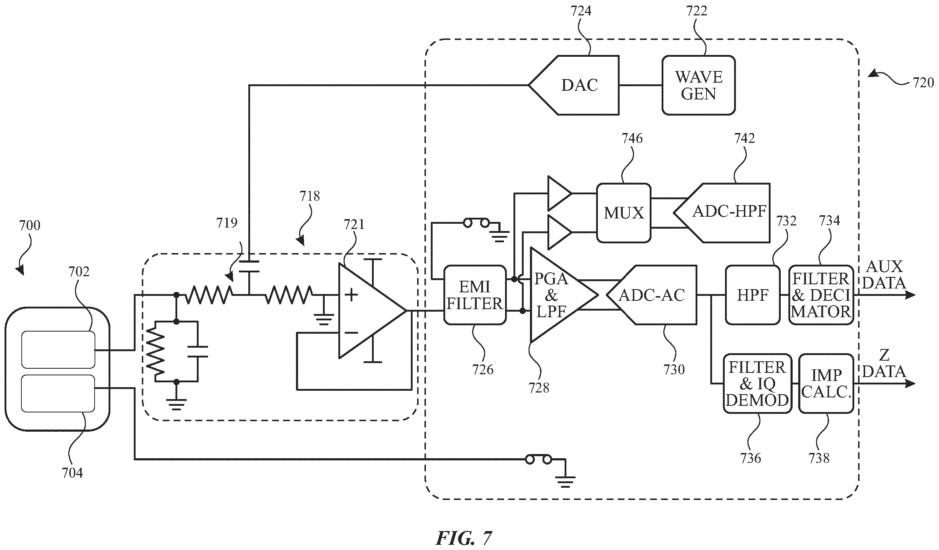

FIG. 7 illustrates an example configuration of circuitry configured to detect suboptimal physiological signal measurement conditions according to examples of the disclosure.

In some examples, the electronic devices described herein are configured to detect suboptimal measurement conditions. For example, when attempting to obtain EMG data, the electronic device can detect that excess moisture and/or dirt has accumulated between the user's skin and measurement electrodes, and/or an excess amount of noise is coupling to the EMG signals causing erroneous EMG data, thus adversely affecting determinations of hand pose and/or movement (e.g., when at least partially relying upon EMG signals). In some examples, the suboptimal measurement environment is determined when conductance between the electrodes 702 and 704 exceeds a threshold value (e.g., 10 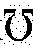 , 100 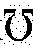 , 1k 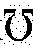 , 10k 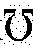 , 100k 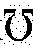 , 1M 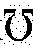 , 10M 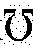 , or 100M 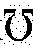 ) and/or when a level of noise exceeds a threshold noise level (e.g., 500, 750, 1000, 2000, 3000, 4000, or 5000 µV). For example, the electronic device optionally grounds a first electrode 704 and performs a conductance measurement using a second electrode 702 (e.g., corresponding to electrodes 302 and/or 304 of FIG. 3). In some examples, electronic device 700 includes stimulation circuitry 720, such as including a stimulation and/or waveform generator 722 coupled to inject a signal via a digital-to-analog converter (DAC) 724 at a node 719 where the second electrode feeds into an input of an amplifier 721 (e.g., the signal is injected at a location where the second electrode 702 is coupled to the amplifier 721 input via one or more filters, signal conditioning circuitry such as impedance dividers, and/or buffers included in signal conditioning circuitry 718). In some examples, the node 719 is coupled to a first input of an amplifier 721 (e.g., a non-inverting input), and a second input of the amplifier 721 (e.g., an inverting input) is coupled to an output of the amplifier 721 to provide negative feedback. In some examples, the output of the amplifier 721 is coupled to a filter 726 (e.g., an electromagnetic interference filter). In some examples, the filter 726 is coupled (e.g., differentially) to another amplifier 728, such as a variable gain amplifier, and/or is coupled to a low-pass filter. The output of the other amplifier 728 and/or the low-pass filter optionally can be coupled to an analog-to-digital converter (ADC) 730, and one or more outputs of the ADC optionally are coupled to further signal conditioning circuitry (e.g., high-pass filter 732 and decimator 734, and/or to filter and IQ demodulator 736 and/or impedance calculator 738) to obtain information and/or data to obtain an indication of impedance and/or conductance between the first and the second electrode 704 and 702, respectively.

In some examples, when the conductance is determined to be greater than the threshold level of conductance (e.g., 10 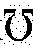 , 100 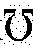 , 1k 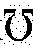 , 10k 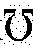 , 100k 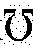 , 1M 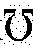 , 10M 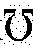 , or 100M 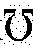 ), the electronic device 700 determines that EMG measurement data may be unreliable, and accordingly can de-prioritize or discard EMG data that would otherwise be used to detect movement and/or poses of the user's body. In some examples, the conductance and/or noise thresholding are evaluated concurrently or separately. In some examples, the conductance and/or noise values are determined over a sliding period of time. For example, the electronic device 700 optionally compares the conductance and/or noise values to respective thresholds where the values are determined periodically, within 0.05, 0.1, 0.5, 1, 1.5, 2, 3, or 5 seconds of determining a current conductance and/or noise sample. In some examples, the electronic device 700 configures the stimulation circuitry 720, switching circuitry, and/or electrodes 702 and 704 to detect conductance and/or detect noise in accordance with a determination that a pose and/or gesture will or is being provided by the user. For example, an operating system included in memory of the electronic device 700 optionally indicates that an application hosted by the electronic device 700 will require movement, and before detecting pose and/or movement, configures the electronic device 700 to determine the quality of the measurement environment. Additionally or alternatively, the fusion module 656 optionally provides data and/or information that suggests that data obtained from EMG module 650 indicates that a user's current pose and/or movement is a first pose and/or movement, and the gyroscopic module 644 and/or optical module 648 indicates that the user's current pose and/or movement is a second, different pose and/or movement. To validate the data obtained from EMG module 650, the electronic device 700 can proactively perform the detection of the measurement quality described herein, and/or update the fusion module 656 with information to determine the reliability and/or weighting of information and/or data sourced from EMG module 650. It is understood that the examples described herein are merely exemplary, and that additional or alternative events can cause evaluation of the measurement quality.

In some examples, when one or both of the conductance and/or noise levels exceed their respective threshold levels, the electronic device can de-prioritize or discard EMG data to detect movement and/or poses of the user's body. Therefore, in addition to detecting EMG and/or ECG signals, the electrodes can be used to determine a suboptimal measurement condition exists for detecting physiological signals (e.g., EMG signals),. Thus, in some examples, in accordance with a determination that a quality of a measurement environment satisfies one or more criteria, the electronic device can perform a physiological measurement to detect a pose and/or gesture of the user's body (e.g., finger(s), hand, and/or arm wearing the electronic device). In some examples, the one or more criteria include criterion that are satisfied when a level of conductance between the electrodes is greater than a threshold level of conductance, and a criterion that is satisfied when a level of noise included in one or more physiological signals (e.g., EMG signals) is greater than a threshold level of noise.

The foregoing description, for purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the invention and its practical applications, to thereby enable others skilled in the art to best use the invention and various described embodiments with various modifications as are suited to the particular use contemplated.

As described above, one aspect of the present technology is the gathering and use of data available from various sources to improve user experiences. The present disclosure contemplates that in some instances, this gathered data may include personal information data that uniquely identifies or can be used to contact or locate a specific person. Such personal information data can include demographic data, location-based data, telephone numbers, email addresses, twitter IDs, home addresses, data or records relating to a user's health or level of fitness (e.g., vital signs measurements, medication information, exercise information), date of birth, or any other identifying or personal information.

The present disclosure recognizes that the use of such personal information data, in the present technology, can be used to the benefit of users. For example, the personal information data can be used to improve user experiences. Further, other uses for personal information data that benefit the user are also contemplated by the present disclosure. For instance, health and fitness data may be used to provide insights into a user's general wellness, or may be used as positive feedback to individuals using technology to pursue wellness goals.

The present disclosure contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining personal information data private and secure. Such policies should be easily accessible by users, and should be updated as the collection and/or use of data changes. Personal information from users should be collected for legitimate and reasonable uses of the entity and not shared or sold outside of those legitimate uses. Further, such collection/sharing should occur after receiving the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and adapted to applicable laws and standards, including jurisdiction-specific considerations. For instance, in the US, collection of or access to certain health data may be governed by federal and/or state laws, such as the Health Insurance Portability and Accountability Act (HIPAA); whereas health data in other countries may be subject to other regulations and policies and should be handled accordingly. Hence different privacy practices should be maintained for different personal data types in each country.

Despite the foregoing, the present disclosure also contemplates embodiments in which users selectively block the use of, or access to, personal information data. That is, the present disclosure contemplates that hardware and/or software elements can be provided to prevent or block access to such personal information data. For example, the present technology can be configured to allow users to select to "opt in" or "opt out" of participation in the collection of personal information data during registration for services or anytime thereafter. In addition to providing "opt in" and "opt out" options, the present disclosure contemplates providing notifications relating to the access or use of personal information. For instance, a user may be notified upon downloading an app that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing specific identifiers (e.g., date of birth, etc.), controlling the amount or specificity of data stored (e.g., collecting location data a city level rather than at an address level), controlling how data is stored (e.g., aggregating data across users), and/or other methods.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data. For example, a user experience can be generated by inferring preferences based on non-personal information data or a bare minimum amount of personal information, such as the content being requested by the device associated with a user, other non-personal information available to the service, or publicly available information.

Therefore, according to the above, some examples of the disclosure are directed to a wearable device. In some examples, the wearable device can comprise a plurality of electrodes, sensing circuitry, pose detection circuitry, different from the sensing circuitry, and one or more processors. In some examples, the one or more processors can be configured to configure the plurality of electrodes and the sensing circuitry in a first configuration to detect first physiological signals corresponding to a first type of physiological measurement using the plurality of electrodes and using the pose detection circuitry in a first mode of operation. In some examples, in a second mode of operation, the one or one or more processors can be configured to configure the plurality of electrodes and the sensing circuitry in a second configuration to detect second physiological signals corresponding to a second type of physiological measurement, different from the first type of physiological measurement, using the plurality of electrodes.

Additionally or alternatively to one or more of the examples disclosed above, in some examples, the first type of physiological measurement can be an electromyogram.

Additionally or alternatively to one or more of the examples disclosed above, in some examples, the second type of physiological measurement can be an electrocardiogram.

Additionally or alternatively to one or more of the examples disclosed above, in some examples, the pose detection circuitry can include one or more inertial measurement units (IMUs).

Additionally or alternatively to one or more of the examples disclosed above, in some examples, the wearable electronic device can further comprise switching circuitry and a plurality of amplifiers including a first amplifier and a second amplifier. In some examples, in the first mode of operation, a first pair of the plurality of electrodes can be differentially coupled to the first amplifier via the switching circuitry, and in the second mode of operation, a second pair of the plurality of electrodes, different from the first pair of the plurality of electrodes, can be differentially coupled to the second amplifier via the switching circuitry.

Additionally or alternatively to one or more of the examples disclosed above, in some examples, the sensing circuitry can include one or more analog-to-digital converters (ADCs) coupled to outputs of the first amplifier and the second amplifier.

Additionally or alternatively to one or more of the examples disclosed above, in some examples, the one or more processors can be further configured to, during the first mode of operation, use one or more machine learning models to detect a movement of a portion of a body of a user wearing the wearable electronic device.

Additionally or alternatively to one or more of the examples disclosed above, in some examples, the portion of the user's body can include a hand of the user wearing the wearable electronic device, and using the one or more machine learning models to detect the movement of the portion of the user's body can include determining a predicted gesture corresponding to movement of the hand.

Additionally or alternatively to one or more of the examples disclosed above, in some examples, processors can be further configured to, during the first mode of operation, use one or more machine learning models to detect a pose of a portion of a body of a user wearing the wearable electronic device.

Additionally or alternatively to one or more of the examples disclosed above, in some examples, the portion of the user's body can include a hand of the user wearing the wearable electronic device, and using the one or more machine learning models to detect the pose of the portion of the user's body can include determining a predicted pose of the hand.

Additionally or alternatively to one or more of the examples disclosed above, in some examples, the wearable electronic device can further comprise stimulation circuitry couplable to one of the plurality of electrodes and the sensing circuitry for injecting a test signal into the sensing circuitry. In some examples, wherein the one or more processors can be further configured to determine a quality of the first physiological measurement from the sensing circuitry based on the injected test signal, and in accordance with a determination that the quality of the first physiological measurement satisfy one or more criteria, detect a movement of a portion of a body of a user wearing the wearable electronic device using the first physiological signals and using the pose detection circuitry, and in accordance with a determination that the quality of the first physiological measurement does not satisfy the one or more criteria, detect the movement of the portion of the user's body using the pose detection circuitry, forgoing use of the first physiological signals to detect the movement.

Additionally or alternatively to one or more of the examples disclosed above, in some examples, the one or more criteria include a criterion that can be satisfied when a conductance between a first electrode of the plurality of electrodes and a second electrode of the plurality of electrodes can be greater than a threshold level of conductance.

Additionally or alternatively to one or more of the examples disclosed above, in some examples, the one or more criteria can include a criterion that can be satisfied when a level of noise associated with the first physiological signals is greater than a threshold level of noise.

Additionally or alternatively to one or more of the examples disclosed above, in some examples, the wearable electronic device can further comprise stimulation circuitry couplable to one of the plurality of electrodes and the sensing circuitry for injecting a test signal into the sensing circuitry. In some examples, the one or more processors can be further configured to determine a quality of the first physiological measurement from the sensing circuitry based on the injected test signal, and in accordance with a determination that the quality of the first physiological measurement satisfy one or more criteria, detect a pose of a portion of a body of a user wearing the wearable electronic device using the first physiological signals and using the pose detection circuitry, and in accordance with a determination that the quality of the first physiological measurement does not satisfy the one or more criteria, detect the pose of the portion of the user's body using the pose detection circuitry, forgoing use of the first physiological signals to detect the movement.

Some examples of the disclosure are directed to a non-transitory computer readable medium storing instructions. In some examples, the one or instructions can be configured to cause one or more processors included in a wearable electronic device including a plurality of electrodes, sensing circuitry, and pose detection circuitry, different from the sensing circuitry to, in a first mode of operation, configure the plurality of electrodes and the sensing circuitry in a first configuration to detect first physiological signals corresponding to a first type of physiological measurement using the plurality of electrodes and using the pose detection circuitry. In some examples, the one or instructions can be configured to cause the one or more processors to, in a second mode of operation, configure the plurality of electrodes and the sensing circuitry in a second configuration to detect second physiological signals corresponding to a second type of physiological measurement, different from the first type of physiological measurement, using the plurality of electrodes.

Some examples of the disclosure are directed to a method of operating a wearable electronic device comprising a plurality of electrodes, sensing circuitry, and pose detection circuitry, different from the sensing circuitry. In some examples, the method can comprise, in a first mode of operation, configuring the plurality of electrodes and the sensing circuitry in a first configuration to detect first physiological signals corresponding to a first type of physiological measurement using the plurality of electrodes and using the pose detection circuitry. In some examples, the method can comprise, in a second mode of operation, configuring the plurality of electrodes and the sensing circuitry in a second configuration to detect second physiological signals corresponding to a second type of physiological measurement, different from the first type of physiological measurement, using the plurality of electrodes.

Some examples of the disclosure are directed to detecting, analyzing, and storing health data. It is understood that health data storage and usage are done so in view of best practices to ensure anonymity and privacy of user data.

Although examples of this disclosure have been fully described with reference to the accompanying drawings, it is to be noted that various changes and modifications will become apparent to those skilled in the art. Such changes and modifications are to be understood as being included within the scope of examples of this disclosure as defined by the appended claims.

Exemplary methods, wearable electronic device, and non-transitory computer-readable storage media are set out in the following items.
1. A method comprising:
   at a wearable device comprising a plurality of electrodes, sensing circuitry, pose detection circuitry, different from the sensing circuitry, and one or more processors:
   in a first mode of operation:
      configuring the plurality of electrodes and the sensing circuitry in a first configuration to detect first physiological signals corresponding to a first type of physiological measurement using the plurality of electrodes and using the pose detection circuitry; and
   in a second mode of operation,
   configuring the plurality of electrodes and the sensing circuitry in a second configuration to detect second physiological signals corresponding to a second type of physiological measurement, different from the first type of physiological measurement, using the plurality of electrodes.
2. The method of item 1, wherein the first type of physiological measurement is an electromyogram and the second type of physiological measurement is an electrocardiogram.
3. The method of any of items 1-2, wherein the pose detection circuitry includes one or more inertial measurement units.
4. The method of any of item 1-3, wherein the wearable electronic device further comprises switching circuity and a plurality of amplifiers including a first amplifier and a second amplifier, wherein:
   in the first mode of operation, a first pair of the plurality of electrodes are differentially coupled to the first amplifier via the switching circuitry, and
   in the second mode of operation, a second pair of the plurality of electrodes, different from the first pair of the plurality of electrodes, are differentially coupled to the second amplifier via the switching circuitry.
5. The method of item 4, wherein the sensing circuitry includes one or more analog-to-digital converters coupled to outputs of the first amplifier and the second amplifier.
6. The method of any of items 1-5, further comprising:
   during the first mode of operation, using one or more machine learning models to detect a movement of a portion of a body of a user wearing the wearable electronic device.
7. The method of item 6, wherein the portion of the body of the user includes a hand of the user wearing the wearable electronic device, and using the one or more machine learning models to detect the movement of the portion of the body of the user includes determining a predicted gesture corresponding to movement of the hand.
8. The method of item 7, wherein the portion of the body of the user includes a hand of the user wearing the wearable electronic device, and using the one or more machine learning models to detect the pose of the portion of the body of the user includes determining a predicted pose of the hand.
9. The method of any of items 1-8, further comprising:
   during the first mode of operation, using one or more machine learning models to detect a pose of a portion of a body of a user wearing the wearable electronic device.
10. The method of any of items 1-9, wherein the wearable electronic device further comprises stimulation circuitry couplable to one of the plurality of electrodes and the sensing circuitry for injecting a test signal into the sensing circuitry, the method further comprising:
   determining a quality of the first physiological signals from the sensing circuitry based on the injected test signal, and
   in accordance with a determination that the quality of the first physiological signals satisfy one or more criteria:
      detecting a movement of a portion of a body of a user wearing the wearable electronic device using the first physiological signals and using the pose detection circuitry; and
   in accordance with a determination that the quality of the first physiological signals does not satisfy the one or more criteria:
      detecting the movement of the portion of the user's body using the pose detection circuitry; and
      forgoing use of the first physiological signals to detect the movement.
11. The method of item 10, wherein the one or more criteria include a criterion that is satisfied when a conductance between a first electrode of the plurality of electrodes and a second electrode of the plurality of electrodes is greater than a threshold level of conductance.
12. The method of any of items 10-11, wherein the one or more criteria include a criterion that is satisfied when a level of noise associated with the first physiological signals is greater than a threshold level of noise.
13. The method of any of items 1-12, wherein the wearable electronic device further comprises stimulation circuitry couplable to one of the plurality of electrodes and the sensing circuitry for injecting a test signal into the sensing circuitry, the method further comprising:
   determining a quality of the first physiological signals from the sensing circuitry based on the injected test signal, and
   in accordance with a determination that the quality of the first physiological signals satisfy one or more criteria:
      detecting a pose of a portion of a body of a user wearing the wearable electronic device using the first physiological signals and using the pose detection circuitry, and
   in accordance with a determination that the quality of the first physiological signals does not satisfy the one or more criteria,
      detecting the pose of the portion of the user's body using the pose detection circuitry; and
      forgoing use of the first physiological signals to detect the pose.
14. A wearable electronic device comprising:
   a plurality of electrodes;
   sensing circuitry;
   pose detection circuitry, different from the sensing circuitry; and
   one or more processors, wherein the one or more processors are configured to perform any one of the methods of items 1-13.
15. A non-transitory computer readable medium storing instructions, wherein the one or instructions are configured to cause one or more processors included in a wearable electronic device further including a plurality of electrodes, sensing circuitry, and pose detection circuitry, different from the sensing circuitry, to perform any one of the methods of items 1-13.

The foregoing description, for purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the invention and its practical applications, to thereby enable others skilled in the art to best utilize the invention and various embodiments with various modifications as are suited to the particular use contemplated.

## Claims

1. A method comprising:
at a wearable device comprising a plurality of electrodes, sensing circuitry, pose detection circuitry, different from the sensing circuitry, and one or more processors:
in a first mode of operation:
configuring the plurality of electrodes and the sensing circuitry in a first configuration to detect first physiological signals corresponding to a first type of physiological measurement using the plurality of electrodes and using the pose detection circuitry; and
in a second mode of operation,
configuring the plurality of electrodes and the sensing circuitry in a second configuration to detect second physiological signals corresponding to a second type of physiological measurement, different from the first type of physiological measurement, using the plurality of electrodes.

2. The method of claim 1, wherein the first type of physiological measurement is an electromyogram and the second type of physiological measurement is an electrocardiogram.

3. The method of any of claims 1-2, wherein the pose detection circuitry includes one or more inertial measurement units.

4. The method of any of claim 1-3, wherein the wearable electronic device further comprises switching circuity and a plurality of amplifiers including a first amplifier and a second amplifier, wherein:
in the first mode of operation, a first pair of the plurality of electrodes are differentially coupled to the first amplifier via the switching circuitry, and
in the second mode of operation, a second pair of the plurality of electrodes, different from the first pair of the plurality of electrodes, are differentially coupled to the second amplifier via the switching circuitry.

5. The method of claim 4, wherein the sensing circuitry includes one or more analog-to-digital converters coupled to outputs of the first amplifier and the second amplifier.

6. The method of any of claims 1-5, further comprising:
during the first mode of operation, using one or more machine learning models to detect a movement of a portion of a body of a user wearing the wearable electronic device.

7. The method of claim 6, wherein the portion of the body of the user includes a hand of the user wearing the wearable electronic device, and using the one or more machine learning models to detect the movement of the portion of the body of the user includes determining a predicted gesture corresponding to movement of the hand.

8. The method of claim 7, wherein the portion of the body of the user includes a hand of the user wearing the wearable electronic device, and using the one or more machine learning models to detect the pose of the portion of the body of the user includes determining a predicted pose of the hand.

9. The method of any of claims 1-8, further comprising:
during the first mode of operation, using one or more machine learning models to detect a pose of a portion of a body of a user wearing the wearable electronic device.

10. The method of any of claims 1-9, wherein the wearable electronic device further comprises stimulation circuitry couplable to one of the plurality of electrodes and the sensing circuitry for injecting a test signal into the sensing circuitry, the method further comprising:
determining a quality of the first physiological signals from the sensing circuitry based on the injected test signal, and
in accordance with a determination that the quality of the first physiological signals satisfy one or more criteria:
detecting a movement of a portion of a body of a user wearing the wearable electronic device using the first physiological signals and using the pose detection circuitry; and
in accordance with a determination that the quality of the first physiological signals does not satisfy the one or more criteria:
detecting the movement of the portion of the user's body using the pose detection circuitry; and
forgoing use of the first physiological signals to detect the movement.

11. The method of claim 10, wherein the one or more criteria include a criterion that is satisfied when a conductance between a first electrode of the plurality of electrodes and a second electrode of the plurality of electrodes is greater than a threshold level of conductance.

12. The method of any of claims 10-11, wherein the one or more criteria include a criterion that is satisfied when a level of noise associated with the first physiological signals is greater than a threshold level of noise.

13. The method of any of claims 1-12, wherein the wearable electronic device further comprises stimulation circuitry couplable to one of the plurality of electrodes and the sensing circuitry for injecting a test signal into the sensing circuitry, the method further comprising:
determining a quality of the first physiological signals from the sensing circuitry based on the injected test signal, and
in accordance with a determination that the quality of the first physiological signals satisfy one or more criteria:
detecting a pose of a portion of a body of a user wearing the wearable electronic device using the first physiological signals and using the pose detection circuitry, and
in accordance with a determination that the quality of the first physiological signals does not satisfy the one or more criteria,
detecting the pose of the portion of the user's body using the pose detection circuitry; and
forgoing use of the first physiological signals to detect the pose.

14. A wearable electronic device comprising:
a plurality of electrodes;
sensing circuitry;
pose detection circuitry, different from the sensing circuitry; and
one or more processors, wherein the one or more processors are configured to perform any one of the methods of claims 1-13.

15. A non-transitory computer readable medium storing instructions, wherein the one or instructions are configured to cause one or more processors included in a wearable electronic device further including a plurality of electrodes, sensing circuitry, and pose detection circuitry, different from the sensing circuitry, to perform any one of the methods of claims 1-13.
